# EUROPÄISCHE PATENTSCHRIFT

(11) **EP 4 325 256 B1**
(45) Veröffentlichungstag und Bekanntmachung des Hinweises auf die Patenterteilung: **21.10.2026**
(21) Anmeldenummer: 22191231.4
(22) Anmeldetag: 19.08.2022
(51) Int. Cl.: G01T 1/24

(54) **RÖNTGENEINRICHTUNG UND VERFAHREN ZUM BETRIEB EINER RÖNTGENEINRICHTUNG**
X-RAY DEVICE AND METHOD FOR OPERATING AN X-RAY DEVICE
DISPOSITIF À RAYONS X ET PROCÉDÉ DE FONCTIONNEMENT D'UN DISPOSITIF À RAYONS X

(43) Veröffentlichungstag der Anmeldung: 21.02.2024
(73) Patentinhaber: Siemens Healthineers AG, 91301 Forchheim (DE)
(72) Erfinder: Göderer, Edgar, 91301 Forchheim (DE); Kreisler, Björn, 91353 Hausen (DE); Wirth, Stefan, 91056 Erlangen (DE)
(74) Vertreter: Siemens Healthineers Patent Attorneys

(56) Entgegenhaltungen:
- JP-A- 2003 209 665
- US-A- 6 037 595

## Beschreibung

Die Erfindung betrifft eine Röntgeneinrichtung mit einer Röntgenquelle, einem Halbleiterdetektor und einer Verarbeitungseinrichtung,
- wobei an der von der Vorderseite abgewandten Rückseite des Halbleiterdetektors in mehreren Bildgebungsbereichen des Halbleiterdetektors jeweils wenigstens eine Bildgebungselektrode angeordnet ist und mehrere Bildgebungsmittel der Röntgeneinrichtung jeweils wenigstens eine der Bildgebungselektroden kontaktieren, um Röntgensignale der Bildgebungselektroden betreffende erste Messwerte zu erfassen,
- wobei die Verarbeitungseinrichtung dazu eingerichtet ist, einen Bilddatensatz in Abhängigkeit der ersten Messwerte zu ermitteln,
- wobei wenigstens eine Zusatzelektrode an der Rückseite des Halbleiterdetektors außerhalb der Bildgebungsbereiche angeordnet ist,
dadurch gekennzeichnet, dass die Verarbeitungseinrichtung (4) dazu eingerichtet ist, in Abhängigkeit der zweiten Messwerte (25) wenigstens eine Komponente (26) der Röntgeneinrichtung (1) zu steuern, wobei die Komponente (26) ein Heizmittel zur Beheizung des Halbleiterdetektors (3) ist.

Daneben betrifft die Erfindung ein Verfahren zum Betrieb einer Röntgeneinrichtung.

Die Nutzung von Halbleiterdetektoren, insbesondere von Einzelphotonendetektoren, in Röntgeneinrichtungen, beispielsweise in Computertomographen, ist an sich bekannt. Bei einer Einzelphotonendetektion wird eine Biasspannung an das Halbleitermaterial des Halbleiterdetektors angelegt, wozu typischerweise eine vollflächige Elektrode an der Vorderseite genutzt wird und an der Rückseite separate Elektroden für einzelne Pixel des Halbleiterdetektors vorgesehen werden. Wird nun durch ein Röntgenphoton ein Elektron-Loch-Paar im Halbleitermaterial erzeugt, wird aufgrund der Biasspannung eine Ladungskaskade ausgelöst und es kann die hieraus resultierende Stromspitze erfasst werden, indem über die Bildgebungselektrode, also die für den jeweiligen Pixel an der Detektorrückseite angebrachte Elektrode, abfließender Ströme über ein jeweiliges Bildgebungsmittel erfasst wird. Die Anzahl der detektierten Stromspitzen beschreibt hierbei, bei nicht zu hohen Röntgenintensitäten, die Anzahl der erfassten Röntgenphotonen, während die Höhe der Stromspitze mit der Photonenenergie des jeweiligen Photons korreliert und somit zur energieaufgelösten Bildgebung herangezogen werden kann.

Um eine Separierung der Detektionsereignisse für die einzelnen Bildpunkte zu erreichen, wird einerseits typischerweise ein Streustrahlenraster genutzt, das vor dem Halbleiterdetektor angeordnet ist. Das Streustrahlenraster kann vollständig aus einem röntgenabsorbierenden Material gebildet sein oder Abschnitte aus röntgenabsorbierendem Material können durch wenig röntgenabsorbierende Abstandshalter, beispielsweise aus Zellulose oder Aluminium, miteinander verbunden sein. Die durch das röntgenabsorbierende Material abgeschirmten Bereiche des Halbleitermaterials können auch als Guard-Ring bezeichnet werden.

Zur besseren Bildpunktseparierung wird häufig gezielt Einfluss auf die Feldverteilung dieser Guard-Ringe genommen. Dies kann durch lokal unterschiedliche Dotierungen des Halbleitermaterials erfolgen. Bevorzugt werden jedoch in den dem röntgenabsorbierenden Material gegenüberliegenden Bereich an der Rückseite des Halbleiterdetektors Zusatzelektroden vorgesehen, die mit einem definierten Potential verbunden werden können, um die Feldverteilung im Guard-Ringbereich zu beeinflussen.

Die gemessenen Ströme für die einzelnen Detektorpixel hängen stark von lokalen Eigenschaften des Halbleiters ab. So führt eine Temperaturerhöhung des Halbleiters zu höheren Dunkelströmen für die erwärmten Detektorpixel und lokale Dotierungsunterschiede, die beispielsweise ebenfalls aus der Röntgenbestrahlung resultieren können, können die gemessenen Stromflüsse ebenfalls beeinflussen. Um eine hohe Bildgebungsqualität zu erreichen, müssen daher die lokalen Eigenschaften des Halbleiters möglichst genau bekannt sein bzw. es muss versucht werden, den Zustand des Halbleiters über die Messung möglichst stabil zu halten.

Hierzu wird bislang einerseits eine Temperierung des Halbleiterdetektors genutzt und andererseits können Dunkelstrommessungen für die einzelnen Detektorpixel durchgeführt werden. Da übliche Dunkelstrommessungen jedoch außerhalb des normalen Messbetriebs, beispielsweise bei abgeschalteter Röntgenröhre bzw. nach Schließen eines Shutters, durchgeführt werden müssen, können hierdurch Änderungen der Halbleitereigenschaften während der Messung, beispielsweise eine lokale Beheizung durch während der Messung fließende Biasströme, nicht berücksichtigt werden und führen somit zu Fehlern in der Bildgebung.

Bei bekannten Ansätzen zur Temperierung des Detektors erfolgt die Beheizung des Detektors zudem auf Basis von Temperaturmessungen und somit über eine relativ träge Regelschleife. Auch dies kann die Bildgebung beeinträchtigen.

Die Druckschrift JP 2003 209665 A offenbart einen Festkörperdetektor und eine Methode, mittels derer ein Offset und ein Gain eines ausgelesenen Bildsignals, welche aus einem Dunkelstrom resultieren können, verbessert korrigiert werden sollen unter Ausnutzung von Pixelsignalen von insensitiven Pixeln.

Die Druckschrift US 6 037 595 A offenbart verschiedenartige Anordnungen von Abschirmungselektroden.

Der Erfindung liegt somit die Aufgabe zugrunde, eine Bildgebung durch eine Röntgeneinrichtung weiter zu verbessern. Bei der verbesserten Bildgebung kann es sich z.B. um die Erfassung eines einzelnen Röntgenbildes handeln oder auch um die Erfassung mehrerer Röntgenprojektionen, beispielsweise im Rahmen einer Computertomographie.

Die Aufgabe wird erfindungsgemäß dadurch gelöst, dass wenigstens ein Stromsensor die Zusatzelektrode oder jeweils wenigstens eine der Zusatzelektroden kontaktiert, um den Stromfluss durch die wenigstens eine Zusatzelektrode betreffende zweite Messwerte zu erfassen.

Die zweiten Messwerte können dazu dienen, einen Zustand des Halbleitersensors zu beschreiben bzw. abzuschätzen. Beispielsweise kann eine Temperatur des Halbleitersensors, ein Alterungszustand, ein Qualitätsmaß, z.B. bezüglich der Dotierungseigenschaften des Halbleitermaterials, oder Ähnliches durch die zweiten Messwerte beschrieben bzw. auf deren Basis ermittelt werden.

Insbesondere beschreiben die ersten Messwerte die räumliche Verteilung der durch den Halbleiterdetektor absorbierten Röntgenstrahlung und somit insbesondere unmittelbar ein Abbild der absorbierten Röntgenstrahlung bzw. des durchstrahlten Objekts, das durch den Bilddatensatz beschrieben werden soll. Der Bilddatensatz kann somit ausschließlich in Abhängigkeit der ersten Messwerte ermittelt werden, es ist jedoch auch möglich, die Ermittlung des Bilddatensatzes zusätzlich in Abhängigkeit der zweiten Bilddaten durchzuführen bzw. einen vorläufigen Bilddatensatz, der ausschließlich von den ersten Messwerten abhängt, auf Basis der zweiten Messwerte zu korrigieren, um Einflüsse des Sensorzustandes auf die Bildgebung zu korrigieren.

Anders ausgedrückt können insbesondere ausschließlich in den Bildgebungsbereichen Bilddaten, also Daten, die die räumliche Verteilung der Röntgenabsorption betreffen, erfasst werden. Der Bereich bzw. die Bereiche, in denen der wenigstens eine Zusatzsensor angeordnet ist, können insbesondere nicht zur Erfassung von Bilddaten, die die Verteilung der absorbierten Röntgenstrahlung betreffen, genutzt werden bzw. geeignet sein, z.B. aufgrund einer Abschirmung, z.B. durch ein Streustrahlenraster, eine Blende oder Ähnliches, oder aufgrund von Dotierungseigenschaften des Halbleitermaterials des Halbleiterdetektors, bzw. nicht für eine solche Erfassung genutzt werden.

Röntgensignale können insbesondere jene Signale, also z.B. an der Elektrode vorhandene Ladungsdichten bzw. diese durchfließende Ströme sein, die durch Absorption von Röntgenquanten in dem der jeweiligen Bildgebungselektrode zugeordneten Bildgebungsbereich verursacht wurden. Die ersten Messwerte können beispielsweise die Häufigkeit bzw. Anzahl und/oder die Höhe von erfassten Stromspitzen betreffen bzw. allgemein wenigstens eine Eigenschaft eines zeitlichen Verlaufs des Stroms durch die Bildgebungselektrode, jedoch z.B. auch Potentialmessungen oder Ähnliches.

Durch zusätzliche Erfassung der zweiten Messwerte werden auch Signale, z.B. Ströme, erfasst, die außerhalb der Bildgebungsbereiche, z.B. in Abschattungsbereichen des Halbleiterdetektors bzw. innerhalb der Guard-Ringstrukturen durch das Halbleitermaterial fließen. Da diese Bereiche zumindest weitgehend gegen Röntgenphotonen der Röntgenquelle abgeschirmt sind, können die durch die zweiten Messwerte gemessenen Ströme als eine Art Dunkelstrom betrachtet werden. Gegenüber üblichen Dunkelstrommessungen wird jedoch der Vorteil erreicht, dass die Messung während des normalen Messvorgangs zur Bildgebung möglich ist und somit beispielsweise auch ein Einfluss einer lokalen Erwärmung des Röntgendetektors durch einfallende Röntgenstrahlung berücksichtigt werden kann.

Insbesondere bei gemeinsamer Auswertung der ersten und zweiten Messwerte und bei Nutzung hinreichend vieler Zusatzelektroden, beispielsweise bei Nutzung einer Zusatzelektrode zwischen jedem Paar von Bildgebungselektroden, kann durch Auswertung der ersten und zweiten Messwerte auch die Bewegung der durch ein jeweiliges Röntgenphoton ausgelösten Ladungswolken in dem Halbleitermaterial genauer erkannt und analysiert werden, so dass beispielsweise auch Zusatzinformationen über die Wechselwirkungen zwischen Bildpunkten ermittelt und bei der Ermittlung des Bilddatensatzes berücksichtigt werden können.

Da somit die im Halbleitermaterial fließenden Ströme aufgrund der Auswertung der zweiten Messwerte genauer bekannt sind, kann beispielsweise die lokale Beheizung des Halbleitermaterials durch solche Ströme mit guter Genauigkeit prädiziert werden, womit es beispielsweise möglich wird, die Beheizung des Halbleitermaterials zur Temperierung des Halbleiterdetektors nicht oder zumindest nicht ausschließlich auf Basis einer erfassten Temperatur durchzuführen, sondern zukünftige Temperaturen aufgrund von Stromflüssen bereits zu prädizieren und somit die obig erläuterte Trägheit der Temperaturstabilisierung zumindest weitgehend zu vermeiden.

Erfindungsgemäß ist die Verarbeitungseinrichtung dazu eingerichtet, in Abhängigkeit der zweiten Messwerte wenigstens eine Komponente der Röntgeneinrichtung zu steuern, wobei die Komponente ein Heizmittel zur Beheizung des Halbleiterdetektors ist. Darüber hinaus können weitere Komponenten der Röntgeneinrichtung in Abhängigkeit der zweiten Messwerte gesteuert werden.

Ergänzend oder alternativ können durch Überwachung der zweiten Messwerte lokale Materialveränderungen, beispielsweise höhere Dunkelströme aufgrund der lokalen Temperatur oder aufgrund von lokalen Defekten im Halbleiter, bereits während der Messung erkannt und daher bei der Auswertung der ersten Messwerte bzw. bei der Ermittlung des Bilddatensatzes berücksichtigt werden. In einem einfachen Beispiel kann davon ausgegangen werden, dass der Strom über die Zusatzelektroden zumindest näherungsweise proportional zum Dunkelstrom ist, wenn dieser, abgesehen von der Einstrahlung der Röntgenstrahlung, unter gleichen Bedingungen ermittelt würde. Somit können an sich bekannte Algorithmen zur Dunkelstromkorrektur in der erfindungsgemäßen Röntgeneinrichtung jedoch auf Basis der zweiten Messwerte parametrisiert bzw. zumindest korrigiert werden, um erst im Rahmen der Messdatenerfassung auftretende Änderungen des lokalen Zustands des Halbleitermaterials bzw. in vorangehenden Messungen ohne dazwischen liegende Dunkelstrommessungen erfolgte Änderungen berücksichtigt zu können. Die resultierende verbesserte Dunkelstromkorrektur kann es beispielsweise ermöglichen, zeitlich nah aufeinander erfolgende Röntgendetektionen im gleichen Bildpunkt besser separieren zu können.

Die Erfassung der zweiten Messwerte kann zudem dazu beitragen, die Gesamtintensität der auf den Halbleiterdetektor eingestrahlten Röntgenstrahlung mit höherer Genauigkeit abzuschätzen, womit es beispielsweise möglich wird, in Abhängigkeit der erfassten Gesamtintensität zwischen verschiedenen Auswertealgorithmen zur Erzeugung des Bilddatensatzes zu wechseln oder Ähnliches.

Wie obig erläutert, kann die Verarbeitungseinrichtung vorteilhaft dazu eingerichtet sein, den Bilddatensatz zusätzlich in Abhängigkeit der zweiten Messwerte zu ermitteln.

Neben der erläuterten erfindungsgemäßen Steuerung einer Heizung des Halbleiterdetektors bzw. der Berücksichtigung der zweiten Messwerte bei der Bilddatenauswertung, können ergänzend auch andere Komponenten der Röntgeneinrichtung in Abhängigkeit der zweiten Messwerte gesteuert werden. Beispielsweise kann eine Blendenstellung oder eine Röhrenspannung angepasst werden, um beispielsweise bei erkannten sehr hohen Gesamtintensitäten die auf den Detektor einfallende Röntgenintensität zu reduzieren. Bei hohen Röntgenintensitäten kann es beispielsweise auch zweckmäßig sein, eine Biasspannung zwischen einer Biaselektrode an der Vorderseite des Halbleiterdetektors und den Bildgebungselektroden zu reduzieren, um ein schnelleres Abklingen der Stromspitzen und somit eine bessere Separierung von kurz nacheinander eintreffenden Röntgenphotonen zu ermöglichen.

Die Röntgeneinrichtung kann ein zwischen der Röntgenquelle und einer der Röntgenquelle zugewandten Vorderseite des Halbleiterdetektors angeordnetes Streustrahlenraster umfasst, wobei wenigstens ein Abschattungsbereich des Halbeiterdetektors durch ein röntgenabsorbierendes Material des Streustrahlenrasters von der Röntgenquelle abgeschirmt ist, wobei die wenigstens eine Zusatzelektrode jeweils zumindest abschnittsweise in dem Abschattungsbereich oder wenigstens einem der Abschattungsbereiche angeordnet ist.

Beispielsweise können Paare von Bildgebungsbereichen jeweils durch den Abschattungsbereich oder wenigstens einen der Abschattungsbereiche voneinander getrennt sein. Hierbei ist es möglich, dass eine Erfassung zweiter Messwerte für Zusatzelektroden zwischen allen Bildgebungsbereichen erfolgt. Es ist jedoch auch möglich, dass zwischen einigen Bildgebungsbereichen keine Zusatzelektroden vorhanden sind bzw. dass dort zwar Elektroden vorhanden sind, die wie in üblichen Halbleiterdetektoren auf einem vorgegebenen Potential gehalten werden, um einen Guard-Ring zu bilden, für diese Elektroden jedoch keine Strommessung erfolgt.

Die jeweilige Zusatzelektrode kann sich jeweils parallel zu einer Strebe des Streustrahlenrasters erstrecken, wobei die Zusatzelektrode insbesondere etwas breiter als diese Strebe ausgebildet sein kann, beispielsweise um den Faktor 2 oder 5 oder 10. Eine an der Vorderseite des Halbleiterdetektors angeordnete Biaselektrode kann durchgehend ausgebildet sein, jedoch auch durch mehrere separate Elektroden gebildet sein. Das Streustrahlenraster kann ausschließlich aus dem röntgenabsorbierenden Material bestehen und beispielsweise durch ein Wolfram- oder Bleigitter gebildet werden. Es ist jedoch möglich, dass in Zwischenbereichen zwischen Abschnitten aus röntgenabsorbierendem Material röntgendurchlässiges Material angeordnet ist, beispielsweise Zellulose oder Aluminium.

Die Verarbeitungseinrichtung der Röntgeneinrichtung kann beispielsweise in einem Gehäuse der Röntgeneinrichtung angeordnet sein, jedoch auch als separate Komponente ausgebildet sein, beispielsweise als Arbeitsplatzrechner oder Server. Es ist auch möglich, dass die Verarbeitungseinrichtung als dezentrales System, beispielsweise als Cloud-Lösung, implementiert ist.

Die Bildgebungselektroden und die wenigstens eine Zusatzelektrode können durch wenigstens ein jeweiliges Kontaktmittel mit einer jeweiligen Verbindungselektrode eines integrierten Schaltkreises verbunden sein, der die Bildgebungsmittel implementiert. Entsprechende Lösungen, bei denen insbesondere ein applikationsspezifischer integrierter Schaltkreis (ASIC) als integrierter Schaltkreis verwendet wird, sind für übliche Halbleiterdetektoren für Röntgeneinrichtungen, bei denen keine Strommessung für die Zusatzelektroden erfolgt, an sich bekannt. Neu ist hingegen die erfindungsgemäße Kontaktierung der Zusatzelektroden zu Strommessung über den integrierten Schaltkreis.

Die Kontaktmittel können insbesondere starre Kontaktmittel sein, beispielsweise Lotperlen bzw. allgemein Metallperlen, die zur Kontaktierung aufgeschmolzen werden können.

Innerhalb des integrierten Schaltkreises kann insbesondere unmittelbar eine Digital-Analog-Wandlung der erfassten Ströme bzw. der diese Ströme betreffenden ersten Messwerte erfolgen, so dass beispielsweise eine digitale Kommunikation zwischen integriertem Schaltkreis und einer separat von diesem ausgebildeter Verarbeitungseinrichtung ausreichend sein kann.

Der integrierte Schaltkreis kann zusätzlich den Stromsensor implementieren. Somit kann die Strommessung im integrierten Schaltkreis selbst erfolgen und insbesondere können auch die zweiten Messwerte unmittelbar digitalisiert werden.

Alternativ wäre es beispielsweise möglich, den zu messenden Strom von den Zusatzelektroden zwar über den integrierten Schaltkreis zu führen, dort jedoch für jeden der Stromsensoren einen zusätzlichen Ausgang des integrierten Schaltkreises bereitzustellen, über den der Strom zum jeweiligen separat ausgebildeten Stromsensor geführt werden kann. Daher kann der Stromsensor separat von dem integrierte Schaltkreis ausgebildet ist, wobei der integrierte Schaltkreis einen jeweiligen Schaltkreisabschnitt aufweist, der dazu dient, den zu messende Strom von der jeweiligen Zusatzelektrode zu dem jeweiligen Stromsensor zu führen

Da integrierte Schaltkreise zur Verarbeitung von Signalen von Halbleiterdetektoren in Röntgeneinrichtungen häufig ohnehin dazu genutzt werden, Elektroden im Abschattungsbereich auf einem definierten Potential zu halten, ist somit nur eine relativ geringfügige Modifikation an solchen bekannten integrierten Schaltkreisen erforderlich, womit die erfindungsgemäße Röntgeneinrichtung mit geringem Aufwand implementiert werden kann.

Die Zusatzelektrode oder wenigstens eine der Zusatzelektroden kann über wenigstens zwei voneinander beabstandete Kontaktmittel mit einer oder mit einer jeweiligen Verbindungselektrode des integrierten Schaltkreises verbunden sein. Hierdurch kann einerseits der aus dieser Verbindung resultierende Widerstand reduziert werden, was vorteilhaft sein kann, da, wie später noch erläutert werden wird, die Zusatzelektroden vorzugsweise trotz der erfolgenden Strommessung möglichst genau auf einem vorgegebenen Potential gehalten werden sollen. Zugleich kann durch Nutzung mehrerer Kontaktmittel erreicht werden, dass einzelne defekte Verbindungen die Funktion des Halbleiterdetektors nicht oder zumindest nur unwesentlich beeinträchtigen, so dass eine höhere Ausbeute bei der Detektorherstellung und eine längere Lebenszeit des Detektors erreicht werden können. Die Nutzung mehrerer Kontaktmittel kann zudem vorteilhaft sein, wenn relativ großflächige Zusatzelektroden genutzt werden sollen, da hierdurch lokale Potentialunterschiede an der Zusatzelektrode minimiert werden können.

Wie bereits erläutert, ist die Verarbeitungseinrichtung dazu eingerichtet, in Abhängigkeit der zweiten Messwerte zumindest ein Heizmittel zur Beheizung des Halbleiterdetektors als die Komponente zu steuern. Wie bereits obig erläutert wurde, ist die Regelung der Heizleistung eines solchen Heizmittels besonders relevant, um möglichst konstante Betriebsbedingungen des Röntgendetektors zu erreichen. Durch Berücksichtigung der zweiten Messwerte können zukünftige Temperaturänderungen aufgrund von Stromflüssen im Halbleiterdetektor prädiziert und somit Regelverzögerungen vermieden bzw. zumindest reduziert werden.

Das Heizmittel kann zur gleichmäßigen Beheizung des Halbleiterdetektors oder auch zur Beheizung einzelner Segmente des Halbleiterdetektors dienen. Können einzelne Segmente des Halbleiterdetektors unabhängig voneinander beheizt werden, ist es besonders vorteilhaft, wenn für jedes der Segmente wenigstens ein separater Stromsensor genutzt wird, um einen Strom über wenigstens eine dortige Zusatzelektrode zu erfassen, um lokale Stromflüsse in den einzelnen Segmenten zu ermitteln und bei der Beheizung zu berücksichtigen.

Die Zusatzelektrode oder jeweils wenigstens eine der Zusatzelektroden kann als ein geradliniger Streifen und/oder als ein einen, insbesondere rechteckigen, Bildgebungsbereich umlaufender Streifen und/oder kreuzförmig ausgebildet sein.

Ein geradliniger Streifen kann sich insbesondere parallel zu einer Strebe des Streustrahlenrasters erstrecken. Besonders bevorzugt erstreckt sich ein solcher Streifen über eine Länge zwischen zwei senkrecht hierzu verlaufenden weiteren Streben des Streustrahlenrasters, so dass er im Wesentlichen eine seitliche Grenze eines Bildgebungsbereichs abdeckt. Alternativ ist es jedoch auch möglich, erheblich kürzere Streifen zu nutzen, um im Wesentlichen punktförmig lokale Ströme zu messen. Durch Nutzung von Streifenelektroden bzw. im Wesentlichen punktförmigen Zusatzelektrode können insbesondere Ströme an verschiedenen Grenzflächen, die einen Bildgebungsbereich gegenüber einem jeweiligen weiteren Bildgebungsbereich abgrenzen, gemessen werden, wodurch beispielsweise Ströme in einem Detektorpixel aufgrund einer Röntgendetektion in einem benachbarten Detektorpixel genauer abgeschätzt werden können.

Die Nutzung eines den Bildgebungsbereich umlaufenden Streifens kann hingegen vorteilhaft sein, um einen lokalen Zustand in eben diesem Bildgebungsbereich besonders genau abschätzen zu können.

Die Nutzung kreuzförmiger Zusatzelektroden kann vorteilhaft sein, um mit einer einzigen Zusatzelektrode beispielsweise lokale Zustände in vier angrenzenden Bildgebungsbereichen abzuschätzen. Dies kann beispielsweise vorteilhaft sein, wenn relativ wenige Zusatzelektroden genutzt werden sollen.

Die Zusatzelektrode und die Bildgebungselektroden können derart elektrisch miteinander oder mit einem gemeinsamen Referenzpotential verbunden sein, dass sich das Potential an den Bildgebungselektroden und das Potential an der wenigstens einen Zusatzelektrode beim Betrieb der Röntgeneinrichtung um maximal 1 Volt oder Maximal 200 mV voneinander unterscheiden.

Die jeweilige Zusatzelektrode und die Bildgebungselektroden können hierzu beispielsweise derart, insbesondere über den jeweiligen Stromsensor bzw. das jeweilige Bildgebungsmittel, mit einem im Betrieb der Röntgeneinrichtung auf einem Referenzpotential liegenden Referenzpunkt verbunden sein, dass der Widerstand zwischen einer an der Vorderseite des Halbleiterdetektors angeordneten Biaselektrode und der jeweiligen Zusatz- bzw. Bildgebungselektrode wenigstens um den Faktor 10 oder 100 größer ist als der Widerstand zwischen dieser Zusatz- bzw. Bildgebungselektrode und dem Referenzpunkt. Der Referenzpunkt kann insbesondere ein Anschluss jenes integrierten Schaltkreises sein, der die Strommessung implementiert.

Durch die niederohmige Verbindung mit dem Referenzpunkt kann erreicht werden, dass die Zusatzelektrode im Wesentlichen auf dem Referenzpotential gehalten werden kann. Insbesondere können Abweichungen von weniger als 0,1 V zum Referenzpotential realisiert werden und somit eine ähnlich genaue Vorgabe des Referenzpotentials, wie sie in Halbleiterdetektoren mit Elektroden im Abschattungsbereich, für die keine Strommessung erfolgt, üblich ist.

Die Verbindung zum Referenzpunkt kann insbesondere niederohmig über den Stromsensor erfolgen.

Mehreren zusammenhängenden Teilbereichen des Halbleiterdetektors, die jeweils wenigstens eine der Bildgebungselektroden umfassen, kann jeweils wenigstens einer der Stromsensoren zugeordnet sein, der wenigstens eine der Zusatzelektroden kontaktiert, die in diesem Teilbereich angeordnet ist, um den Stromfluss durch diese Zusatzelektrode betreffende zweite Messwerte zu erfassen. Es können somit ortsaufgelöst für mehrere Teilbereiche Stromflüsse in mehreren Abschattungsbereichen erfasst werden, um beispielsweise eine lokale Erwärmung des Halbleiterdetektors bzw. lokale Materialveränderungen erkennen und berücksichtigen zu können.

Neben der erfindungsgemäßen Röntgeneinrichtung betrifft die Erfindung ein Verfahren zum Betrieb einer Röntgeneinrichtung, die eine Röntgenquelle und einen Halbleiterdetektor umfasst, wobei an der von der Vorderseite abgewandten Rückseite des Halbleiterdetektors in mehreren außerhalb des Abschattungsbereichs liegenden Bildgebungsbereichen des Halbleiterdetektors jeweils wenigstens eine Bildgebungselektrode angeordnet ist, wobei wenigstens eine Zusatzelektrode an der Rückseite des Halbleiterdetektors außerhalb der Bildgebungsbereiche angeordnet ist, umfassend die Schritte:
- Erfassen von Röntgensignale der Bildgebungselektroden betreffenden ersten Messwerten,
- Erfassen von den Stromfluss durch die wenigstens eine Zusatzelektrode betreffenden zweiten Messwerten, und
- Ermitteln eines Bilddatensatzes in Abhängigkeit der ersten Messwerte,
- Ansteuern wenigstens einer Komponente (26) der Röntgeneinrichtung (1) in Abhängigkeit der zweiten Messwerte (25), wobei die Komponente (26) ein Heizmittel zur Beheizung des Halbleiterdetektors (3) ist.

Das erfindungsgemäße Verfahren kann mit den zur erfindungsgemäßen Röntgeneinrichtung erläuterten Merkmalen mit den dort genannten Vorteilen weitergebildet werden und umgekehrt.

Die Erfassung der ersten Messwerte kann insbesondere nach der Erfassung der zweiten Messwerte und der Steuerung der Komponente erfolgen. Beispielsweise können die zweiten Messwerte jeweils während einer vorangehenden Röntgenaufnahme erfasst werden, beispielsweise bei einer Computertomographie während einer vorangehend erfassten Projektionsaufnahme.

Ergänzend oder alternativ ist es möglich, auch während einer einzelnen Bilderfassung bzw. während einer kontinuierlichen Bilderfassung, insbesondere mit niedriger Intensität, die Ansteuerung der Komponenten der Röntgeneinrichtung dynamisch in Abhängigkeit der zweiten Messwerte anzupassen.

Weitere Vorteile und Einzelheiten der Erfindung ergeben sich aus den folgenden Ausführungsbeispielen sowie den zugehörigen Zeichnungen. Hierbei zeigen schematisch:
- Fig. 1: ein Ausführungsbeispiel einer erfindungsgemäßen Röntgeneinrichtung,
- Fig. 2: ein Ablaufdiagramm eines Ausführungsbeispiels des erfindungsgemäßen Verfahrens, und
- Fig. 3: eine beispielhafte Anordnung von Bildgebungs- und Zusatzelektroden an einem Halbleitersensor in einem weiteren Ausführungsbeispiel einer erfindungsgemäßen Röntgeneinrichtung.

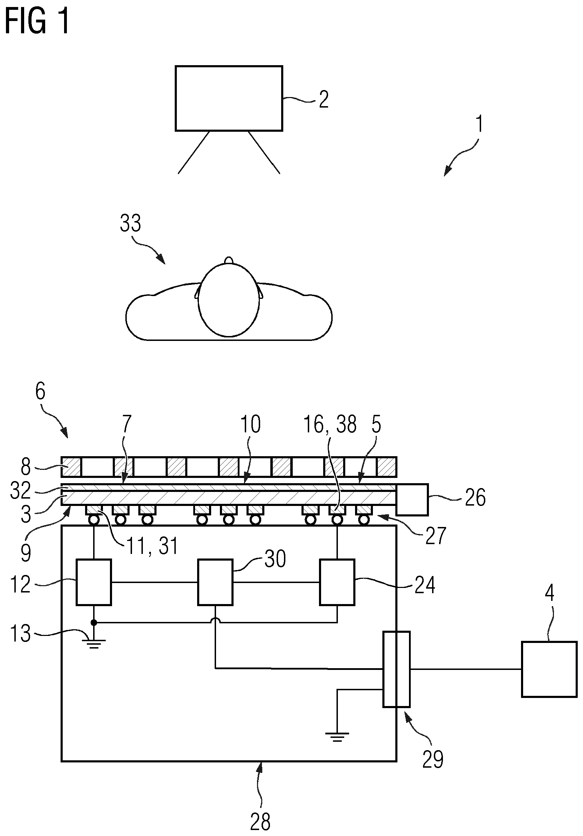

Fig. 1 zeigt schematisch eine Röntgeneinrichtung 1 mit einer Röntgenquelle 2 und einem Halbleiterdetektor 3, durch die im Beispiel ein Bilddatensatz bezüglich eines Untersuchungsobjekts 33, z. B. eines Patienten, erfasst werden soll. Die Röntgeneinrichtung 1 kann beispielsweise ein Computertomograph oder auch eine Vorrichtung zur Aufnahme einzelner zweidimensionaler Röntgenbilder sein.

Zur klareren Darstellung der erfindungsrelevanten Merkmale sind die Größenverhältnisse zwischen den verschiedenen Komponenten in Fig. 1 deutlich verzerrt dargestellt. Insbesondere sind der Halbleiterdetektor 3, der mit diesem verbundene integrierte Schaltkreis 28 und das zwischen dem Halbleiterdetektor 3 und der Röntgenquelle 2 angeordnete Streustrahlenraster 6 verglichen mit dem Untersuchungsobjekt 33 bzw. der Röntgenquelle 2 deutlich vergrößert dargestellt. Aus Übersichtlichkeitsgründen wird zudem ein Halbleiterdetektor 3 mit relativ wenigen Bildgebungsbereichen 10 bzw. Pixeln dargestellt, wobei in realen Halbleiterdetektoren mehrere hundert Bildgebungsbereiche pro Zeile implementiert werden können.

In der Röntgeneinrichtung 1 wird eine an sich bekannte Einzelphotonendetektion genutzt. Hierbei wird an das Halbleitermaterial des Halbleiterdetektors 3 eine Biasspannung angelegt, in dem eine im Beispiel durchgehende Biaselektrode 32 an der Vorderseite 5 des Halbleiterdetektors 3 mit einer Biasspannung beaufschlagt wird und die an der Rückseite in den Bildgebungsbereichen 10 angeordneten Bildgebungselektroden 11 über Bildgebungsmittel 12 mit einem Referenzpotential 13 gekoppelt werden. Durch geeignete Wahl der Biasspannung resultiert bei Erzeugung eines Elektronen-Loch-Paars im Halbleitermaterial eine Ladungskaskade, die als Spannungspuls über erste Messwerte des Bildgebungsmittels 12 erfasst werden kann.

Zur besseren Separierung der Bildpixel ist zwischen dem Halbleiterdetektor 3 und der Röntgenquelle 2 im Beispiel ein Streustrahlenraster 6 angeordnet, dessen röntgenabsorbierendes Material 8 die hinter den Streben des Streustrahlenrasters 6 liegenden Abschattungsbereiche 7 des Halbleiterdetektors 3 abschirmt. Es ist hierbei bekannt, an der Rückseite 9 des Halbleiterdetektors 3 in diesen Abschattungsbereichen 7 bzw. allgemein außerhalb der Bildgebungsbereiche 10 Zusatzelektroden 16 vorzusehen, die mit einem Referenzpotential 13 beaufschlagt werden.

Im Gegensatz zu üblichen Röntgeneinrichtungen wird in der in Fig. 1 gezeigten Röntgeneinrichtung jedoch wenigstens ein Stromsensor 24 genutzt, der wenigstens eine der Zusatzelektroden 16 kontaktiert, um den Stromfluss durch die kontaktierte Zusatzelektrode 16 bzw. die kontaktierten Zusatzelektroden 16 betreffende zweite Messwerte zu erfassen.

Wie bereits im allgemeinen Teil erläutert wurde, kann durch Strommessung in den Abschattungsbereichen 7 einerseits eine Art Dunkelstrommessung selbst während der Messung selbst erfolgen. Andererseits kann, insbesondere bei lokal aufgelöster Erfassung von zweiten Messwerten durch Nutzung separater Stromsensoren 24 für die, wie in Fig. 1 gezeigt, verteilt angeordneten Zusatzelektroden, eine Bewegung von Ladungswolken im Halbleitermaterial genauer abgeschätzt werden. Hierdurch können lokale Veränderungen des Halbleitermaterials erkannt bzw. Temperaturveränderungen aufgrund von im Halbleitermaterial fließenden Strömen prädiziert werden und somit der aktuelle und zukünftige Zustand des Halbleiterdetektors genauer bestimmt werden.

Erfindungsgemäß wird dies genutzt, um zumindest ein Heizmittel zur Beheizung des Halbleiterdetektors 3 zu steuern, im Beispiel nur schematisch dargestellt. Entsprechende Heizmittel werden in üblichen Röntgeneinrichtungen auf Basis einer gemessenen Halbleiterdetektortemperatur gesteuert, wodurch Regelungsverzögerungen resultieren können. Die genauere Erfassung von Stromflüssen im Halbleiterdetektor 3 mithilfe der zweiten Messwerte ermöglicht es hingegen in der gezeigten Messeinrichtung, eine zukünftige Erwärmung des Halbleitermaterials bereits abzuschätzen, bevor diese erfolgt, womit das Heizmittel bzw. die Komponente 26 mit erheblich geringerer Verzögerung oder sogar verzögerungsfrei angesteuert werden kann, um beispielsweise bei starken Stromflüssen im Halbleitermaterial 3 die extern zugeführte Heizleistung zu reduzieren, um eine Beheizung des Halbleitermaterials durch die internen Stromflüsse zu kompensieren.

Darüber hinaus können die zweiten Messwerte genutzt werden, um einen zu ermittelnden Bilddatensatz in Abhängigkeit der ersten und zweiten Messwerte, also der Messwerte bezüglich Bildgebungselektroden und bezüglich Zusatzelektroden, zu ermitteln. Insbesondere können die zweiten Messwerte und somit die ermittelten Stromflüsse im Abschattungsbereich 7 herangezogen werden, um eine an sich bekannte Dunkelstrommessung bei inaktiver Röntgenquelle 2 zu ersetzen oder zu korrigieren. Somit können an sich bekannte Ansätze zur Dunkelstromsubtraktion im Rahmen der Bilddatenverarbeitung nun zusätzlich die zweiten Messwerte berücksichtigen, um beispielsweise Veränderungen eines Dunkelstroms durch eine lokale Beheizung bereits im laufenden Betrieb während der Messung zu erkennen und bei der Bilddatenverarbeitung zu kompensieren.

Die Ermittlung der ersten Messwerte und somit die Erfassung der bildgebenden Stromspitzen und die Ermittlung der zweiten Messwerte wird im Beispiel durch einen integrierten Schaltkreis 28, beispielsweise einen applikationsspezifischen integrierten Schaltkreis, implementiert, dessen nicht gezeigte Verbindungselektroden über ein jeweiliges Kontaktmittel 27, beispielsweise eine Lot- bzw. Metallperle, mit den Bildgebungselektroden 11 und den Zusatzelektroden 16 verbunden sind. Im Rahmen der Herstellung kann beispielsweise der integrierte Schaltkreis 28 mit den daran angeformten Kontaktmitteln 27 auf den Halbleiterdetektor 3 aufgesetzt und durch Anschmelzen der Kontaktmittel 27 sowohl elektrisch als auch mechanisch mit diesem verbunden werden.

Die ersten und zweiten Messwerte können durch die Bildgebungsmittel bzw. Stromsensoren digital erfasst und über eine Kommunikationsschnittstelle 30, beispielsweise eine serielle Schnittstelle, an die Verarbeitungseinrichtung 4 übertragen werden. Die Verbindung erfolgt im Beispiel über die Steckverbindung 29, über die auch das Referenzpotential 13 bereitgestellt werden kann. Hierbei sind die Verbindung der Zusatzelektroden 16 über die Kontaktmittel 27 und den jeweiligen Stromsensor 24 zum Referenzpotential 13 und die Verbindung der Bildgebungselektroden 11 über die Kontaktmittel 27 und das jeweilige Bildgebungsmittel 24 zum Referenzpotential 13 hinreichend niederohmig bzw. zumindest ähnlich resistiv, dass das Potential 38 an der Zusatzelektrode 16 um maximal 0,1 V von dem Potential 31 an den Bildgebungselektroden abweicht. Hierzu kann der Widerstand dieser niederohmigen Verbindung beispielsweise um wenigstens einen Faktor 100 geringer sein als der durch das Halbleitermaterial 3 verursachte Widerstand zwischen der Zusatzelektrode 16 und der Biaselektrode 32.

Zur Widerstandsreduzierung und/oder zur Verbesserung der Fehlertoleranz im Rahmen der Herstellung des Halbleiterdetektors bzw. zur Erhöhung von dessen Lebenszeit kann es zweckmäßig sein, die jeweiligen Zusatzelektroden 16 jeweils über mehrere voneinander beabstandete der Kontaktmittel 27 zu verbinden. Hierbei kann für jedes der Kontaktmittel 27 eine separate Verbindungselektrode des integrierten Schaltkreises 28 vorgesehen sein oder die verschiedenen Kontaktmittel 27 können die gleiche Verbindungselektrode kontaktieren.

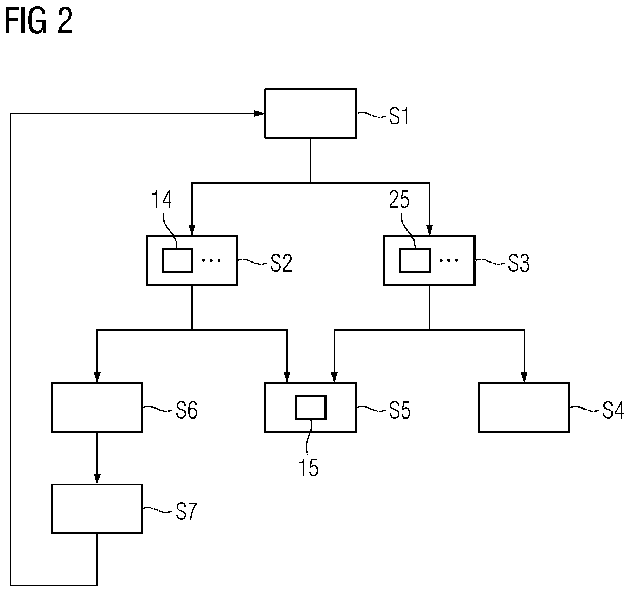

Fig. 2 zeigt ein Ablaufdiagramm eines möglichen Verfahrens zum Betrieb der in Fig. 1 gezeigten Röntgeneinrichtung 1. Hierbei wird in Schritt S1 die Röntgenquelle 2 aktiviert, beispielsweise durch Bestromung einer Röntgenröhre bzw. durch Öffnen eines Shutters.

In Schritt S2 werden über die Bildgebungsmittel 12 jeweilige erste Messwerte 14 erfasst, die jeweils Röntgensignale an wenigstens einer mit dem jeweiligen Bildgebungsmittel 12 verbundene Bildgebungselektrode 11 betreffen. Hierbei ist insbesondere für jeden Bildpunkt des ermittelten Bilddatensatzes 15 wenigstens ein Bildgebungsmittel 12 vorgesehen, um Röntgenintensitäten in dem diesem Bildpunkt zugeordneten Bildgebungsbereich 10 des Halbleiterdetektors 3 zu erfassen. Aus Übersichtlichkeitsgründen ist in Fig. 1 nur einer dieser Bildgebungsmittel 12 dargestellt.

In Schritt S3 werden, insbesondere parallel zu Schritt S2, über wenigstens einen Stromsensor 24 Ströme über jeweils wenigstens eine Zusatzelektrode 16 als zweite Messwerte 25 erfasst. Vorzugsweise erfolgt auch diese Messung ortsaufgelöst, womit mehrere Stromsensoren 24 genutzt werden können, von denen aus Übersichtlichkeitsgründen in Fig. 1 nur einer dargestellt ist.

In Schritt S4 werden die zweiten Messwerte 25 ausgewertet, um die Komponente 26, erfindungsgemäß eine Heizeinrichtung für den Halbleiterdetektor 3, zu steuern. Dies und eine mögliche Steuerung anderer relevanter Komponenten wurde bereits obig erläutert.

Während es prinzipiell möglich ist, eine entsprechende Ansteuerung unmittelbar durchzuführen, kann es zweckmäßig sein, zunächst das Ende einer aktuellen Bildgebung abzuwarten und eine entsprechende Ansteuerung der Komponenten erst in einem vorbereiteten Schritt für eine nachfolgende Bildaufnahme, beispielsweise vor der Aufnahme einer weiteren Projektion im Rahmen einer Computertomographie, durchzuführen.

In Schritt S5 wird ein Bilddatensatz 15 ermittelt. Im Beispiel erfolgt diese Ermittlung sowohl in Abhängigkeit der ersten Messwerte 14 als auch in Abhängigkeit der zweiten Messwerte 25. Beispielsweise können anhand der zweiten Messwerte 25 Dunkelströme für mehrere Abschnitte des Halbleiterdetektors 3 abgeschätzt werden, die im Rahmen einer Dunkelstromkorrektur bei der Verarbeitung der ersten Messwerte 14, die ansonsten in üblicher Weise erfolgen kann, berücksichtigt werden können.

Obwohl in dem gezeigten Beispiel sowohl die Ansteuerung einer Komponente 26 in Abhängigkeit der zweiten Messwerte 25 in Schritt S4 als auch die Berücksichtigung der zweiten Messwerte 25 bei der Ermittlung des Bilddatensatzes 15 erfolgt, sind auch Ausgestaltungen des beschriebenen Verfahrens möglich, bei denen die zweiten Messwerte 25 nur auf eine der beiden beschriebenen Weisen verwendet werden.

In Schritt S6 wird die Röntgenquelle 2 nach Erfassung der ersten und zweiten Messwerte 14, 25 deaktiviert und die Erfassung des aktuellen Röntgenbildes bzw. eine aktuelle Projektion bei der Erfassung von Computertomographiedaten ist somit beendet. In Schritt S7 kann nun beispielsweise eine Repositionierung der Röntgenquelle 2 und des Halbleiterdetektors 3 sowie der damit verbundenen Komponenten erfolgen, um das Untersuchungsobjekt 33 beispielsweise aus einem anderen Blickwinkel abzubilden, wonach das Verfahren ab Schritt S1 wiederholt werden kann.

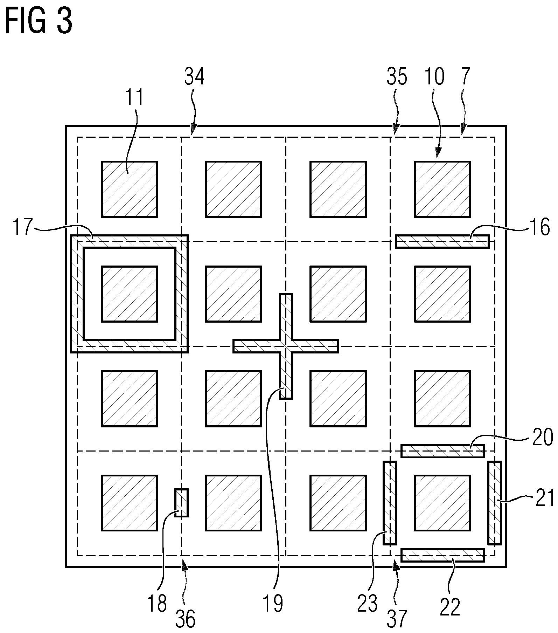

Fig. 3 zeigt eine Draufsicht auf die Rückseite eines Halbleiterdetektors, der in der vorangehend erläuterten Röntgeneinrichtung verwendet werden kann. Aus Übersichtlichkeitsgründen ist wiederum ein Halbleiterdetektor mit sehr wenigen Bildgebungsbereichen 10 gezeigt, im Beispiel mit sechszehn Bildgebungsbereichen 10. Die Bildgebungsbereiche 10 sind durch den gitterförmigen Abschattungsbereich 7 voneinander getrennt, der durch die Form der Streben des nicht gezeigten Streustrahlenrasters vorgegeben wird.

In jedem der Bildgebungsbereiche 10 ist eine jeweilige Bildgebungselektrode 11 angeordnet. Durch Messung des durch die jeweilige Bildgebungselektrode 11 fließen Stroms, insbesondere durch Zählen von Stromspitzen, kann eine in dem jeweiligen Bildbereich 10 einfallende Röntgenintensität ermittelt werden, womit eine Stromspitzenanzahl pro Zeit beispielsweise als Bildwert eines dem Bildgebungsbereich 10 zugeordneten Bildpunktes genutzt werden kann.

Im gezeigten Beispiel werden eine Vielzahl verschieden geformter Zusatzelektroden 16 - 23 genutzt, für die beispielsweise jeweils separat eine Erfassung von zweiten Messwerten über einen jeweiligen zugeordneten Stromsensor erfolgen kann. Die Nutzung sehr vieler verschiedener Zusatzelektrodenformen an einem einzigen Halbleiterdetektor erfolgt primär zu Illustrationszwecken. In real umgesetzten Halbleiterdetektoren werden typischerweise nur ein oder zwei verschiedene Formen der Zusatzelektroden 16 - 23 genutzt werden, wobei es in einigen Fällen jedoch vorteilhaft sein kann, auch mehr verschiedene Formen zu nutzen.

Die Zusatzelektrode 16 ist streifenförmig und erstreckt sich parallel zu einer Strebe des Streustrahlenrasters zwischen zwei Bildgebungsbereichen 10. Eine derartige Ausgestaltung kann vorteilhaft sein, um beispielsweise Stromflüsse zwischen Bildgebungsbereichen 10 und somit eine gegenseitige Beeinflussung von Bildpunkten besser zu erkennen und potentiell zu korrigieren.

Die Zusatzelektrode 17 umläuft streifenförmig einen Bildgebungsbereich 10 vollständig. Dies kann vorteilhaft sein, um robust und mit geringem Aufwand lokale Eigenschaften des umfassten Bildgebungsbereichs 10 zu erfassen, beispielsweise einen dort zu erwartenden Dunkelstrom, und diese beispielsweise bei der Bilddatenauswertung zu berücksichtigen bzw. zur Anpassung einer Heizungssteuerung zu nutzen.

Wie für die Zusatzelektrode 18 gezeigt ist, kann eine Stromerfassung auch nur für einen kurzen Abschnitt des Zwischenbereichs zwischen zwei Bildgebungsbereichen 10 erfolgen, beispielsweise, um durch Nutzung mehrerer solcher Zusatzelektroden eine höhere Ortsauflösung für die Ermittlung der zweiten Messwerte zu erreichen.

Die Kreuzform der Zusatzelektrode 19 ermöglicht es durch eine einzelne Elektrode mit guter Genauigkeit lokale Eigenschaften in allen vier benachbarten Bildgebungsbereichen 10 zu erkennen und zu berücksichtigen.

Die Zusatzelektroden 20 - 23 entsprechen in ihrer Form der Zusatzelektrode 16, sind jedoch umlaufend um einen Bildgebungsbereich 10 gruppiert. Werden alle vier Zusatzelektroden 20 - 23 mit einem gemeinsamen Stromsensor kontaktiert, resultiert ein ähnliches Messergebnis wie bei der Nutzung der Zusatzelektrode 17.

Werden hingegen separate zweite Messwerte für die vier Zusatzelektroden 20 - 23 ermittelt, können sowohl, wie zur Zusatzelektrode 16 erläutert, Stromflüsse zwischen benachbarten Bildgebungsbereichen 10 analysiert werden, als auch die Eigenschaften im Bildgebungsbereich 10 besonders genau abgeschätzt werden, wie zur Zusatzelektrode 17 erläutert wurde. Nachteilig kann bei einer solchen Ausgestaltung jedoch sein, dass relativ viele Kontakte zu einem integrierten Schaltkreis erforderlich sind, wenn eine solche Anordnung von Zusatzelektroden 20 - 23 für alle oder zumindest für sehr viele Bildgebungsbereiche 10 genutzt werden soll, weshalb auch die anderen erläuterten Zusatzelektrodenformen zweckmäßig sein können.

Der in Fig. 3 gezeigte Halbleiterdetektor weist vier Quadranten bzw. Teilbereiche 34 - 36 auf, in denen jeweils vier Bildgebungsbereiche 10 angeordnet sind und die jeweils wenigstens eine der Zusatzelektroden 16 - 23 aufweisen. Erfolgt für die Zusatzelektroden 16 - 23 jeweils eine separate Erfassung von zweiten Messwerten über einen jeweiligen zugeordneten Stromsensor, können somit die Eigenschaften des Halbleiterdetektors, beispielsweise seine Temperatur oder vorhandene Defekte bzw. deren Auswirkungen auf die Messung, separat für die einzelnen Quadranten berücksichtigt werden, da eine ortsaufgelöste Erfassung der zweiten Messwerte erfolgen kann. Hierdurch können auch lokale Effekte kompensiert werden, beispielsweise indem ein lokal unterschiedliches Beheizen des Halbleiterdetektors erfolgt und/oder indem in unterschiedliche Bereichen unterschiedliche Dunkelstromkorrekturen genutzt werden, wenn die zweiten Messwerte für die unterschiedlichen Teilbereiche 34 - 37 unterschiedliche Eigenschaften bzw. Zustände des Halbleitermaterials anzeigen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgeneinrichtung mit einer Röntgenquelle (2), einem Halbleiterdetektor (3) und einer Verarbeitungseinrichtung (4),
- wobei an der von der Vorderseite (5) abgewandten Rückseite (9) des Halbleiterdetektors (3) in mehreren Bildgebungsbereichen (10) des Halbleiterdetektors (3) jeweils wenigstens eine Bildgebungselektrode (11) angeordnet ist und mehrere Bildgebungsmittel (12) der Röntgeneinrichtung (1) jeweils wenigstens eine der Bildgebungselektroden (11) kontaktieren, um Röntgensignale der Bildgebungselektroden (11) betreffende erste Messwerte (14) zu erfassen,
- wobei die Verarbeitungseinrichtung (4) dazu eingerichtet ist, einen Bilddatensatz (15) in Abhängigkeit der ersten Messwerte (14) zu ermitteln,
- wobei wenigstens eine Zusatzelektrode (16-23) an der Rückseite (9) des Halbleiterdetektors (3) außerhalb der Bildgebungsbereiche (10) angeordnet ist,
- wobei wenigstens ein Stromsensor (24) die Zusatzelektrode (16-23) oder jeweils wenigstens eine der Zusatzelektroden (16-23) kontaktiert, um den Stromfluss durch die wenigstens eine Zusatzelektrode (16-23) betreffende zweite Messwerte (25) zu erfassen,
**dadurch gekennzeichnet, dass** die Verarbeitungseinrichtung (4) dazu eingerichtet ist, in Abhängigkeit der zweiten Messwerte (25) wenigstens eine Komponente (26) der Röntgeneinrichtung (1) zu steuern, wobei die Komponente (26) ein Heizmittel zur Beheizung des Halbleiterdetektors (3) ist.

2. Röntgeneinrichtung nach Anspruch 1, **dadurch gekennzeichnet, dass** die Verarbeitungseinrichtung (4) dazu eingerichtet ist, den Bilddatensatz (15) zusätzlich in Abhängigkeit der zweiten Messwerte (25) zu ermitteln.

3. Röntgeneinrichtung nach einem der vorangehenden Ansprüche, **dadurch gekennzeichnet, dass** sie ein zwischen der Röntgenquelle (2) und einer der Röntgenquelle (2) zugewandten Vorderseite (5) des Halbleiterdetektors (3) angeordnetes Streustrahlenraster (6) umfasst, wobei wenigstens ein Abschattungsbereich (7) des Halbeiterdetektors (3) durch ein röntgenabsorbierendes Material (8) des Streustrahlenrasters (6) von der Röntgenquelle (2) abgeschirmt ist, wobei die wenigstens eine Zusatzelektrode (16-23) jeweils zumindest abschnittsweise in dem Abschattungsbereich (7) oder wenigstens einem der Abschattungsbereiche (7) angeordnet ist.

4. Röntgeneinrichtung nach einem der vorangehenden Ansprüche, **dadurch gekennzeichnet, dass** die Bildgebungselektroden (11) und die wenigstens eine Zusatzelektrode (16-23) durch wenigstens ein jeweiliges Kontaktmittel (27) mit einer jeweiligen Verbindungselektrode eines integrierten Schaltkreises (28) verbunden sind, der die Bildgebungsmittel (12) implementiert.

5. Röntgeneinrichtung nach Anspruch 4, **dadurch gekennzeichnet, dass** der integrierte Schaltkreis (28) zusätzlich den Stromsensor (24) implementiert.

6. Röntgeneinrichtung nach Anspruch 4, **dadurch gekennzeichnet, dass** der Stromsensor (24) separat von dem integrierten Schaltkreis (28) ausgebildet ist, wobei der integrierte Schaltkreis einen jeweiligen Schaltkreisabschnitt aufweist, der dazu dient, den zu messende Strom von der jeweiligen Zusatzelektrode zu dem jeweiligen Stromsensor (24) zu führen.

7. Röntgeneinrichtung nach einem der Ansprüche 4 bis 6, **dadurch gekennzeichnet, dass** die die Zusatzelektrode (16-23) oder wenigstens eine der Zusatzelektroden (16-23) über wenigstens zwei voneinander beabstandete Kontaktmittel (27) mit einer oder einer jeweiligen der Verbindungselektroden des integrierten Schaltkreises (28) verbunden ist.

8. Röntgeneinrichtung nach einem der vorangehenden Ansprüche, wobei die Zusatzelektrode (16-23) oder jeweils wenigstens eine der Zusatzelektroden (16-23) als ein geradliniger Streifen und/oder als ein einen, insbesondere rechteckigen, Bildgebungsbereich (10) umlaufender Streifen und/oder kreuzförmig ausgebildet ist.

9. Röntgeneinrichtung nach einem der vorangehenden Ansprüche, **dadurch gekennzeichnet, dass** die Zusatzelektrode (16-23) und die Bildgebungselektroden derart elektrisch miteinander oder mit einem gemeinsamen Referenzpotential (13) verbunden sind, dass sich das Potential (31) an den Bildgebungselektroden (11) und das Potential (38) an der wenigstens einen Zusatzelektrode (16-23) beim Betrieb der Röntgeneinrichtung um maximal 1 Volt oder Maximal 200 mV voneinander unterscheiden.

10. Röntgeneinrichtung nach einem der vorangehenden Ansprüche, **dadurch gekennzeichnet, dass** mehreren zusammenhängenden Teilbereichen (34-37) des Halbleiterdetektors (3), die jeweils wenigstens eine der Bildgebungselektroden (11) umfassen, jeweils wenigstens einer der Stromsensoren (24) zugeordnet ist, der wenigstens eine der Zusatzelektroden (16-23) kontaktiert, die in diesem Teilbereich (34-37) angeordnet ist, um den Stromfluss durch diese Zusatzelektrode (16-23) betreffende zweite Messwerte (25) zu erfassen.

11. Verfahren zum Betrieb einer Röntgeneinrichtung (1), die eine Röntgenquelle (2) und einen Halbleiterdetektor (3) umfasst, wobei an der von der Vorderseite (5) abgewandten Rückseite (9) des Halbleiterdetektors (3) in mehreren außerhalb des Abschattungsbereichs (7) liegenden Bildgebungsbereichen (10) des Halbleiterdetektors (3) jeweils wenigstens eine Bildgebungselektrode (11) angeordnet ist, wobei wenigstens eine Zusatzelektrode (16-23) an der Rückseite (9) des Halbleiterdetektors (3) außerhalb der Bildgebungsbereiche angeordnet ist, umfassend die Schritte:
- Erfassen von Röntgensignale der Bildgebungselektroden (11) betreffenden ersten Messwerten (14),
- Erfassen von den Stromfluss durch die wenigstens eine Zusatzelektrode (16-23) betreffenden zweiten Messwerten (25), und
- Ermitteln eines Bilddatensatzes (15) in Abhängigkeit der ersten Messwerte (14),
- Ansteuern wenigstens einer Komponente (26) der Röntgeneinrichtung (1) in Abhängigkeit der zweiten Messwerte (25), wobei die Komponente (26) ein Heizmittel zur Beheizung des Halbleiterdetektors (3) ist.

## Claims

1. X-ray facility having an X-ray source (2), a semiconductor detector (3) and a processing facility (4),
- wherein on the rear side (9) of the semiconductor detector (3) facing away from the front side (5), in each of a plurality of imaging regions (10) of the semiconductor detector (3), at least one imaging electrode (11) is arranged and a plurality of imaging means (12) of the X-ray facility (1) each contact at least one of the imaging electrodes (11) in order to acquire first measurement values (14) relating to X-ray signals of the imaging electrodes (11),
- wherein the processing facility (4) is designed to establish an image dataset (15) dependent upon the first measurement values (14),
- wherein at least one additional electrode (16-23) is arranged on the rear side (9) of the semiconductor detector (3) outside the imaging regions (10),
- wherein at least one current sensor (24) contacts the additional electrode (16-23) or at least one of the additional electrodes (16-23) in each case in order to acquire the current flow by way of the second measurement values (25) relating to the at least one additional electrode (16-23),
**characterised in that** the processing facility (4) is designed to control at least one component (26) of the X-ray facility (1) dependent upon the second measurement values (25), wherein the component (26) is a heating means for heating the semiconductor detector (3).

2. X-ray facility according to claim 1, **characterised in that** the processing facility (4) is designed to establish the image dataset (15) additionally dependent upon the second measurement values (25).

3. X-ray facility according to one of the preceding claims, **characterised in that** it comprises an anti-scatter grid (6) arranged between the X-ray source (2) and a front side (5) of the semiconductor detector (3) facing towards the X-ray source (2), wherein at least one shading region (7) of the semiconductor detector (3) is screened from the X-ray source (2) by an X-ray absorbent material (8) of the anti-scatter grid (6), wherein in each case, the at least one additional electrode (16-23) is arranged at least partially in the shading region (7) or at least one of the shading regions (7).

4. X-ray facility according to one of the preceding claims, **characterised in that** the imaging electrodes (11) and the at least one additional electrode (16-23) are each connected by way of at least one respective contact means (27) to a respective connecting electrode of an integrated circuit (28) which implements the imaging means (12).

5. X-ray facility according to claim 4, **characterised in that** the integrated circuit (28) additionally implements the current sensor (24).

6. X-ray facility according to claim 4, **characterised in that** the current sensor (24) is configured separate from the integrated circuit (28), wherein the integrated circuit has a circuit portion which serves to feed the current to be measured from each additional electrode to the respective current sensor (24).

7. X-ray facility according to one of claims 4 to 6, **characterised in that** the additional electrode (16-23) or at least one of the additional electrodes (16-23) is connected, via at least two contact means (27) spaced apart from one another, to one or each to one of the connecting electrodes of the integrated circuit (28).

8. X-ray facility according to one of the preceding claims, wherein the additional electrode (16-23) or, in each case, at least one of the additional electrodes (16-23) is designed as a straight strip and/or as a strip surrounding an, in particular rectangular, imaging region (10), and/or is cruciform.

9. X-ray facility according to one of the preceding claims, **characterised in that** the additional electrode (16-23) and the imaging electrodes are electrically connected to one another or to a common reference potential (13) such that the potential (31) on the imaging electrodes (11) and the potential (38) on the at least one additional electrode (16-23) differ from one another during operation of the X-ray facility by not more than 1 volt or not more than 200 mV.

10. X-ray facility according to one of the preceding claims, **characterised in that** a plurality of contiguous subregions (34-37) of the semiconductor detector (3) each of which comprises at least one of the imaging electrodes (11) are each associated with at least one of the current sensors (24) which contacts at least one of the additional electrodes (16-23) which is arranged in this subregion (34-37), in order to acquire the current flow by way of second measurement values (25) relating to this additional electrode (16-23).

11. Method for operating an X-ray facility (1) which comprises an X-ray source (2) and a semiconductor detector (3), wherein on the rear side (9) of the semiconductor detector (3) facing away from the front side (5), at least one imaging electrode (11) is arranged in each of a plurality of imaging regions (10) of the semiconductor detector (3) which lie outside of the shading region (7), wherein at least one additional electrode (16-23) is arranged on the rear side (9) of the semiconductor detector (3) outside the imaging regions, comprising the steps:
- acquiring first measurement values (14) relating to X-ray signals of the imaging electrodes (11),
- acquiring second measurement values (25) relating to the current flow through the at least one additional electrode (16-23), and
- establishing an image dataset (15) dependent upon the first measurement values (14)
- actuating at least one component (26) of the X-ray facility (1) dependent upon the second measurement values (25), wherein the component (26) is a heating means for heating the semiconductor detector (3).

## Revendications

1. Dispositif à rayons X comprenant une source (2) de rayons X, un détecteur (3) à semiconducteur et un dispositif (4) de traitement,
- dans lequel, sur la face (9) arrière, non tournée vers la face (5) avant, du détecteur (3) à semiconducteur est disposée, dans plusieurs zones (10) d'imagerie du détecteur (3) à semiconducteur, respectivement au moins une électrode (11) d'imagerie et plusieurs moyens (12) d'imagerie du dispositif (1) à rayons X sont en contact respectivement avec au moins l'une des électrodes (11) d'imagerie, afin de détecter des premières valeurs (14) de mesure concernant des signaux des électrodes (11) d'imagerie,
- dans lequel le dispositif (4) de traitement est agencé pour déterminer un ensemble (15) de données d'image en fonction des premières valeurs (14) de mesure,
- dans lequel au moins une électrode (16-23) supplémentaire est disposée sur la face (9) arrière du détecteur (3) à semiconducteur à l'extérieur des zones (10) d'imagerie,
- dans lequel au moins un capteur (24) de courant est en contact avec l'électrode (16-23) supplémentaire ou respectivement au moins l'une des électrodes (16-23) supplémentaires, afin de détecter des deuxièmes valeurs (25) de mesure concernant le flux de courant passant dans la au moins une électrode (16-23) supplémentaire,
**caractérisé en ce que** le dispositif (4) de traitement est agencé pour commander, en fonction des deuxièmes valeurs (25) de mesure, au moins un composant (26) du dispositif (1) à rayons X, dans lequel le composant (26) est un moyen de chauffage pour chauffer le détecteur (3) à semiconducteur.

2. Dispositif à rayons X suivant la revendication 1, **caractérisé en ce que** le dispositif (4) de traitement est agencé pour déterminer l'ensemble (15) de données d'image en fonction supplémentairement des deuxièmes valeurs (25) de mesure.

3. Dispositif à rayons X suivant l'une des revendications précédentes, **caractérisé en ce qu'**il comprend une trame (6) de rayons de dispersion, disposée entre la source (2) de rayons X et une face (5) avant, tournée vers la source (2) de rayons X, du détecteur (3) à semiconducteur, dans lequel au moins une zone (7) d'assombrissement du détecteur (3) à semiconducteur est protégée de la source (2) de rayons X par un matériau (8) absorbant les rayons X de la trame (6) de rayons de dispersion, dans lequel la au moins une électrode (16-23) supplémentaire est disposée respectivement, au moins par endroits, dans la zone (7) d'assombrissement ou du moins de l'une des zones (7) d'assombrissement.

4. Dispositif à rayons X suivant l'une des revendications précédentes, **caractérisé en ce que** les électrodes (11) d'imagerie et la au moins une électrode (16-23) supplémentaire sont, par au moins un moyen (27) de contact respectif, reliées à une électrode respective de liaison d'un circuit (28) intégré, qui met en œuvre le moyen (12) d'imagerie.

5. Dispositif à rayons X suivant la revendication 4, **caractérisé en ce que** le circuit (28) intégré met en œuvre supplémentairement le capteur (24) de courant.

6. Dispositif à rayons X suivant la revendication 4, **caractérisé en ce que** le capteur (24) de courant est constitué séparément du circuit (28) intégré, dans lequel le circuit intégré a une partie respective de circuit intégré, qui sert à conduire le courant à mesurer de l'électrode supplémentaire respective au capteur (24) de courant respectif.

7. Dispositif à rayons X suivant l'une des revendications 4 à 6, **caractérisé en ce que** l'électrode (16-23) supplémentaire ou du moins l'une des électrodes (16-23) supplémentaire est, par au moins deux moyens (27) de contact à distance l'un de l'autre, reliée à une ou à l'une respective des électrodes de liaison du circuit (28) intégré.

8. Dispositif à rayons X suivant l'une des revendications précédentes, **caractérisé en ce que** l'électrode (16-23) supplémentaire ou respectivement du moins l'une des électrodes (16-23) supplémentaires est constituée sous la forme d'une bande en ligne droite et/ou sous la forme de bandes, en particulier rectangulaires, entourant la zone (10) d'imagerie et/ou en forme de croix.

9. Dispositif à rayons X suivant l'une des revendications précédentes, **caractérisé en ce que** l'électrode (16-23) supplémentaire et les électrodes d'imagerie sont connectées électriquement entre elles ou à un potentiel (13) de référence commun, **en ce que** le potentiel (31) aux électrodes (11) d'imagerie et le potentiel (38) à la au moins une électrode (16-23) supplémentaire sont, lorsque le dispositif à rayons X fonctionne, différents l'un de l'autre de 1 volt au maximum ou de 200 mV au maximum.

10. Dispositif à rayons X suivant l'une des revendications précédentes, **caractérisé en ce que**, à plusieurs zones (34-37) partielles connexes du détecteur (3) à semiconducteur, qui comprennent respectivement au moins l'une des électrodes (11) d'imagerie, est affecté respectivement au moins l'un des capteurs (24) de courant, qui contacte au moins l'une des électrodes (16-23) supplémentaires, qui est disposée dans cette zone (34-37) partielle, afin de détecter des deuxièmes valeurs (25) de mesure concernant le flux de courant passant dans cette électrode (16-23) supplémentaire.

11. Procédé pour faire fonctionner un dispositif (1) à rayons X, qui comprend une source (2) de rayons X et un détecteur (3) à semiconducteur, dans lequel, sur la face (9) arrière, non tournée vers la face (5) avant, du détecteur (3) à semiconducteur, est disposée, en plusieurs zones (10) d'imagerie du détecteur (3) à semiconducteur se trouvant à l'extérieur de la zone (7) d'assombrissement, respectivement au moins une électrode (11) d'imagerie, dans lequel au moins une électrode (16-23) supplémentaire est disposée sur la face (9) arrière du détecteur (3) à semiconducteur à l'extérieur des zones d'imagerie, comprenant les stades :
- détection de premières valeurs (14) de mesure concernant des signaux de rayons X des électrodes (11) d'imagerie,
- détection de deuxièmes valeurs (25) de mesure concernant le flux de courant passant dans la au moins une électrode (16-23) supplémentaire, et
- établissement d'un ensemble (15) de données d'image en fonction des premières valeurs (14) de mesure,
- commande d'au moins un composant (26) du dispositif (1) à rayons X en fonction des deuxièmes valeurs (25) de mesure, dans lequel le composant (26) est un moyen de chauffage pour chauffer le détecteur (3) à semiconducteur.
